# EUROPÄISCHE PATENTSCHRIFT

(11) **EP 2 892 863 B1**
(45) Veröffentlichungstag und Bekanntmachung des Hinweises auf die Patenterteilung: **21.03.2018**
(21) Anmeldenummer: 13753628.0
(22) Anmeldetag: 28.08.2013
(51) Int. Cl.: C07C 1/20, C07C 6/04

(54) **VERFAHREN ZUR HERSTELLUNG VON LINEAREN BUTENEN AUS METHANOL**
METHOD FOR PRODUCING LINEAR BUTENES FROM METHANOL
PROCÉDÉ DE FABRICATION DE BUTÈNES LINÉAIRES À PARTIR DE MÉTHANOL

(30) Priorität: 05.09.2012 DE 102012215757
(43) Veröffentlichungstag der Anmeldung: 15.07.2015
(73) Patentinhaber: Evonik Degussa GmbH, 45128 Essen (DE)
(72) Erfinder: WINTERBERG, Markus, 45731 Waltrop (DE); PETTIJOHN, Ted M, Magnolia, Texas 77354 (US); BOWERS, Stephen, Aldershot Hampshire GU12 6HP (GB); SCHALLENBERG, Jörg, 46286 Dorsten (DE); NAEEM, Shahbaz, 45768 Marl (DE); BUSCH, Oliver Markus, 45665 Recklinghausen (DE)
(86) Internationale Anmeldenummer: PCT/EP2013/067768
(87) Internationale Veröffentlichungsnummer: WO 2014/037254

(56) Entgegenhaltungen:
- US-A- 5 990 369

## Beschreibung

Die Erfindung betrifft ein Verfahren zur Herstellung von linearen Butenen aus Methanol.

Lineare, ungesättigte C₄-Kohlenwasserstoffe wie 1-Buten, cis-2-Buten und trans-2-Buten sind begehrte Ausgangsstoffe und Bausteine für eine Vielzahl von chemischen Produkten. Dazu gehören beispielsweise Polymere wie Polybuten oder Polyethylen, bei welchem 1-Buten als Comonomer für LDPE oder HDPE eingesetzt wird. Des Weiteren werden die Butene im Wege der Hydroformylierung und anschließender Hydrierung zu Weichmacheralkoholen wie Isononanol (INA) oder 2-Propylheptanol (2PH) weiter verarbeitet. Anschließende Veresterung führt zu PVC-Weichmachern wie Diisononylphthalat (DINP) oder Di-2-Propylhepylphthalat.

Die heute verwendeten C₄-Kohlenwasserstoffe werden fast ausschließlich aus Erdöl gewonnen, entweder durch Steam-Cracken (Crack-C4) oder Fluid-katalytisches Cracken (FCC). Die Raffinerien betreiben ihre Cracker aber in der Regel entsprechend dem hohen Bedarf an den C₂-Kohlenwasserstoffen Ethen und Propen; die Ströme an höheren Kohlenwasserstoffen stellen dabei lediglich Zwangsanfälle dar. Die Verfügbarkeit der C₄-Kohlenwasserstoffe hängt damit mit Entwicklungen auf anderen Märkten zusammen. Durch zunehmenden Markterfolg von Produkten, die auf Materialien basieren, die aus dem Ende der Verwertungskette der Butenen stammen, gibt es mittlerweile einen derartig großen Bedarf an Butenen, dass dieser aus petrochemischer Quelle kaum noch gedeckt werden kann. Diese Entwicklung zwingt zur Erschließung neuer Rohstoffquellen.

Eine sehr vorteilhaft erscheinende Option stellen dabei Synthesewege dar, bei denen aus Methanol durch chemische Umsetzung Olefine gewonnen werden. Diese Routinen werden gemeinhin als "Methanol to Olefin Process" (MTO) bezeichnet.

MTO -Verfahren sind in der Patentliteratur bekannt aus WO01/92190A1, EP0448000B1 und DE19723363A1. Bei diesen Verfahren ist die Ausbeute an linearen Butenen aber eher gering; so beträgt beispielsweise bei dem in EP0448000B1 offenbarten Verfahren die Butenausbeute - bezogen auf die Gesamtmenge an erzeugten Kohlenwasserstoffen - nur etwa 30 %. Das in DE19723363A1 beschriebene Verfahren ist hingegen auf maximale Propenausbeute optimiert, sodass nur geringe Mengen an C₄-Kohlenwasserstoffen zu erwarten sind. Eine Übersicht kommerziell betriebener MTO-Verfahren findet sich in Process Economics Programm Report 261 METHANOL TO OLEFINS, erschienen im November 2007, erhältlich bei SRI Consulting. URL: http://www.ihs.com/products/chemical/technology/pep/methanol-to-olefins.aspx

Ein spezielles MTO-Verfahren ist der Methanol-to-Propylene process (MTP-Process), bei welchem Methanol in zwei Reaktionsschritte zunächst in Dimethylether (DME) und dieser sodann in Propen und andere Olefine umgesetzt wird. Der MTP-Prozess wird von LURGI kommerziell angeboten. Eine genauere Beschreibung des LURGI-MTP-Prozesses findet sich im besagten PEP-Report in Abschnitt 5.1, Seite 5-9. Der MTP-Prozess liefert das C₃-Olefin Propen, aber kaum C₄-Olefine. US5990369 beschreibt ein Verfahren zur Herstellung von C2-C4 Olefinen umfassend ein MTO-Verfahren zur Herstellung einer Mischung von C1-C5 Olefinen, die in einer propenreichen und einer propenarmen Fraktion aufgearbeitet wird. Im Lichte dieses Standes der Technik liegt der vorliegenden Erfindung die Aufgabe zu Grunde ein Verfahren zur Herstellung von linearen Butenen aus Methanol anzugeben, bei welchem das eingesetzte Methanol zu einem möglichst großen Anteil in Butene umgesetzt wird.

Gelöst wird die Aufgabe durch ein Verfahren nach Anspruch 1.

Gegenstand der Erfindung ist mithin ein Verfahren zur Herstellung von linearen Butenen aus Methanol umfassend die Schritte a bis f:
a) Bereitstellen von Methanol;
b) Umsetzen des bereitgestellten Methanols in einer ersten Reaktionsstufe in ein erstes Reaktionsgemisch enthaltend Dimethylether, Wasser und gegebenenfalls unumgesetzes Methanol;
c) Umsetzen von Dimethylether in einer zweiten Reaktionsstufe in ein zweites Reaktionsgemisch enthaltend Propen sowie weitere Kohlenwasserstoffe mit zwei, vier und fünf Kohlenstoffatomen, wobei die zweite Reaktionsstufe zumindest teilweise mit dem ersten Reaktionsgemisch gespeist wird;
d) Aufarbeiten des zweiten Reaktionsgemisches unter Erhalt einer propenreichen Fraktion sowie mindestens einer propenarmen Fraktion;
e) Umsetzen von Propen in einer dritten Reaktionsstufe in ein drittes Reaktionsgemisch enthaltend Ethen sowie lineare Butene ausgewählt aus der Gruppe umfassend 1-Buten, cis-2-Buten, trans-2-Buten, wobei die dritte Reaktionsstufe zumindest teilweise mit oder aus der propenreichen Fraktion gespeist wird;
f) Aufarbeiten des dritten Reaktionsgemisches in eine Zielfraktion reich an linearen Butenen und in eine ethenreiche Fraktion,
dadurch gekennzeichnet,
dass die propenarme Fraktion teilweise in die zweite Reaktionsstufe zurückgefahren wird,
dass die propenreiche Fraktion Propan enthält, dass die Umsetzung in der dritten Reaktionsstufe in der Gegenwart von Propan erfolgt und dass im Zuge der Aufarbeitung des dritten Reaktionsgemisches eine propanreiche Fraktion gewonnen wird. Dieses Verfahren beruht auf der Idee, den MTP-Prozess zu kombinieren mit einer dritten Reaktionsstufe in Gestalt einer Metathese, in welcher das aus dem MTP-Prozess erhaltene Propen in die gewünschten linearen Butene (1-Buten, cis-2-Buten und trans-2-Buten) umgesetzt wird.

Technisch vorteilhafter ist es, die dritte Reaktionsstufe mit einer propanhaltigen propenreichen Fraktion zu speisen, sodass die Metathese in Gegenwart von Propan erfolgt. Propan ist in der Metathese inert. In dieser Variante wird das Propan erst nach Durchführung des dritten Reaktionsschrittes aus dem dritten Reaktionsgemisch als propanreiche Fraktion gewonnen. Dies hat den entscheidenden Vorteil, dass die Propan-Propen-Trennung mit einem deutlich geringeren Massendurchsatz durchgeführt werden muss, da das Propen in der vorgeschalteten Metathese zu C₄-Kohlenwasserstoffen umgesetzt wird. Die Trennung der leicht siedenden C₃-Fraktion enthaltend das Propan von der propenhaltigen C₄-Fraktion kann dann deutlich wirtschaftlicher erfolgen.

Für das in der Metathese entstehende Ethen, welches in der ethenreichen Fraktion erhalten wird, sieht die Erfindung wiederum zwei alternative

Verwendungsmöglichkeiten vor: Zum Einen ist es möglich, die ethenreiche Fraktion zumindest teilweise in die zweite Reaktionsstufe (den MTP-Reaktor) zurückzuführen. Das Ethen wird auf diese Weise in Richtung Propen umgesetzt.

Alternativ kann das Ethen auch einer separaten, vierten Reaktionsstufe zugeführt werden, in welcher ein viertes Reaktionsgemisch umfassend lineare Butene aus der Gruppe enthaltend 1-Buten, cis-2-Buten und trans-2-Buten erhaltem wird. Bei der vierten Reaktionsstufe handelt es sich um eine Dimerisierung. Durch die separat durchgeführte Dimerisierung des Ethens in der vierten Reaktionsstufe ist eine weitere Steigerung der Buten-Ausbeute möglich. Auch kann die Dimerisierung selektiver durchgeführt werden als die komplexe Reaktion im MTP-Reaktor (zweite Reaktionsstufe).

Es ist auch möglich, nur einen Teil die ethenreiche Fraktion in die zweite Reaktionsstufe zurückzuführen und den anderen Teil in die Dimerisierung zu verbringen.

In einer bevorzugten Ausführungsform der Erfindung umfasst der Schritt der Aufarbeitung des aus dem MTP-Reaktor stammenden zweiten Reaktionsgemisches die Gewinnung einer Fraktion reich an Kohlenwasserstoffen mit zwei Kohlenstoffatomen, einer Fraktion reich an Kohlenwasserstoffen mit vier Kohlenstoffatomen und eine Fraktion reich an Kohlenwasserstoffen mit fünf Kohlenstoffatomen. Die Aufarbeitung erfolgt destillativ durch Verschaltung einer Mehrzahl von Destillationskolonnen, welche die jeweilige Cₓ-Fraktion abtrennen.

Im Zuge der Aufarbeitung werden somit eine C₂-Fraktion, eine propenreiche C₃-Fraktion, eine C₄-Fraktion und eine C₅-Fraktion erhalten. Die C₂-Fraktion enthält überwiegend Ethen, welches bevorzugt in die zweite Reaktionsstufe (MTP-Reaktor) zurückgeführt wird. Die C₅-Fraktion kann ebenfalls zumindest teilweise zurück in die zweite Reaktionsstufe gefahren werden. Es empfiehlt sich jedoch, einen Teil der C₅-Kohlenwasserstoffe als Purge-Strom aus dem System auszuschleusen, um einer unbilligen Aufkonzentrierung von Hochsiedern entgegenzuwirken.

Der besondere Vorteil dieser komplexen Aufarbeitung besteht darin, dass zugleich eine C₄-Fraktion gewonnen wird, welche die begehrten Butene enthält. Die Butene fallen nämlich nicht nur in der Metathese (dritte Reaktionsstufe) an, sondern bereits auch in dem MTP-Reaktor (zweite Reaktionsstufe). Diese im MTP-Reaktor anfallenden C₄-Kohlenwasserstoffe werden im Wege der aufwendigeren Aufarbeitung noch vor der Metathese gewonnen. Beide C₄-Ströme können selbstverständlich miteinander vermischt und gemeinsam weiter aufgearbeitet werden.

Die im Zuge der Aufarbeitung des zweiten Reaktionsgemisches sollte des Weiteren eine Hochsiederfraktion gewonnen werden, welche die Kohlenwasserstoffe mit mehr als fünf Kohlenstoffatomen enthält. Derartige C₅₊-Kohlenwasserstoffe werden gemeinhin als Hochsieder bezeichnet und neigen in Kreisprozessen dazu sich aufzukonzentrieren. Um die Reaktion nicht unnötig damit zu belasten, empfiehlt es sich, die Hochsiederfraktion aus dem Prozess auszuschleusen.

Im ersten Reaktionsschritt fällt neben dem Dimethylether (DME) noch Wasser an. Dieses Reaktionswasser wird bevorzugt ebenfalls im Zuge der Aufarbeitung des zweiten Reaktionsgemisches als wässrige Fraktion gewonnen und aus dem Prozess ausgeschleust. Das Entwässern des zweiten Reaktionsgemisches erfolgt bevorzugt mittels einer Quensche. Dazu kann auch eine organische Phase abgeschieden werden, welche unreagiertes Methanol und DME enthält. Die organische Phase wird zurück in die zweite Reaktionsstufe gefahren.

Das eingesetzte Methanol kann unterschiedlicher Herkunft sein. Die Basischemikalie Methanol wird als Commodity gehandelt und daher einfachstenfalls zugekauft. Darüber hinaus ist es möglich, das Methanol direkt am Einsatzort oder entfernt davon herzustellen. Die Eigenherstellung des Methanols bietet sich insbesondere dann an, wenn das erfindungsgemäße MTO-Verfahren an einem Verbundstandort betrieben wird. Die Herstellung des Methanols geschieht nach den Verfahrensschritten h bis j:
h) Bereitstellen einer wasserstoffhaltigen oder wasserstofffreien Kohlenstoffquelle;
i) Herstellen von Synthesegas enthaltend Kohlenstoffmonoxid und Wasserstoff aus der Kohlenstoffquelle; erforderlichenfalls unter Zugabe von Wasser bzw. Wasserdampf;
j) katalytisches Umsetzen des Synthesegases in Methanol in einer fünften Reaktionsstufe.

Dies sind übliche Schritte der Methanol-Herstellung. Einzelheiten der Methanol-Herstellung sind bei Ullmann nachzulesen: Fiedler, E., Grossmann, G., Kersebohm, D. B., Weiss, G. and Witte, C. 2011. Methanol. Ullmann's Encyclopedia of Industrial Chemistry.

Als Kohlenstoffquelle kann entweder eine traditionelle fossile Kohlenstoffquelle eingesetzt werden oder ein nachwachsender Rohstoff. Es können auch Mischungen von fossilen und nachwachsenden Kohlenstoffquellen eingesetzt werden. Als Kohlenstoffquelle eignen sich insbesondere Steinkohlen, Braunkohlen, Erdölfraktionen, Ölsand, Erdgas oder Shale-Gas. Dies sind fossile Quellen. Nachwachsende Kohlenstoffquellen können in Gestalt von Holz, Biogas, Biomasse, Müll, Mist oder Klärschlamm erschlossen werden. Als Biogas wird in der Regel eine Gasmischung enthaltend Methan verstanden, die auf biologischem Wege produziert wurde. Am Übergang zwischen fossilen und regenerativen Kohlenstoffquellen liegt der Torf. Besonders bevorzugt wird jedoch Shale-Gas zur Herstellung des Methanols eingesetzt. Dabei handelt es sich um Methan-Gas, welches in Schiefer-Lagerstätten gebunden ist und mittels Einpressens von Chemikalien aufgeschlossen wird (fracking).

Die erste Reaktionsstufe (DME-Synthese) erfolgt bevorzugt in Gegenwart eines festen Silica-Alumina-Katalysators. Diese Reaktion wird bevorzugt in der Dampfphase, also mit gasförmigem Methanol durchgeführt. Die DME-Synthese ist im Stand der Technik bekannt und beispielsweise in EP0448000B1 und DE19723363A1 beschrieben. Hier finden sich auch Einzelheiten zum Katalysator und zu den Reaktionsbedingungen.

Die zweite Reaktionsstufe (MTP-Reaktion), bei welcher das Methanol-Wasser-Dimethylether-Gemisch aus der ersten Reaktionsstufe gegebenenfalls unter Beimischung von aus hinteren Verfahrensschritten rückgeführten Kohlenwasserstoffen zu Olefinen umgesetzt wird, erfolgt vorzugsweise an einem formselektiven Zeolith-Katalysator. Auch bezüglich dieses Schrittes wird auf den allgemeinen Stand der Technik verwiesen, wie er beispielsweise in EP0448000B1 oder in DE19723363A1 dokumentiert ist.

In der dritten Reaktionsstufe wird Propen bzw. ein Propen/Propan-Gemisch (beides als propenreiche Fraktion bezeichnet) an einem Katalysator in der Metathese-Reaktion zumindest teilweise zu linearen Butenen und Ethen umgesetzt. Dies erfolgt im Allgemeinen entsprechend der vereinfachten Reaktionsgleichung:

2 C₃ → 1 C₄ + 1 C₂

Als Metathese-Katalysatoren können beispielsweise heterogene Wolfram- und/oder Molybdän-Katalysatoren eingesetzt werden, wie sie unter anderem in US3340322A oder US3261879A beschrieben sind.

Nicht umgesetztes Propen kann in einer bevorzugten Weiterbildung nach der dritten Reaktionsstufe abgetrennt und in die Metathese zurückgeführt werden. Das durch die Metathese entstandene Ethen wird als ethenreiche Fraktion gewonnen und sodann hochrein aufgearbeitet und als weiteres Verkaufsprodukt vermarktet oder in die zweite Reaktionsstufe zurückgeführt. Die in der Metathese entstandenen linearen Kohlenwasserstoffe sind eine Mischung im Wesentlichen aus 1-Buten, cis-2-Buten und trans-2-Buten. Je nach den gewählten Reaktionsbedingungen und dem Katalysatorsystem kann aber die Ausbeute an 1-Buten oder aber die Ausbeute an 2-Butenen überwiegen.

In einer weiteren Verfahrensvariante kann das in der Metathese entstandene Ethen in einer vierten Reaktionsstufe zu gewünschtem Buten dimerisiert werden. Diese Reaktion läuft beispielsweise mit hoher Selektivität in Gegenwart eines katalytischen Systems aus Trialkylaluminium und Alkyltitanat an Ethern wie beispielsweise Tetrahydrofuran ab. Die Selektivität dieser Reaktion zu 1-Buten lässt sich dadurch steigern, kann der Ether zu der zubereiteten Mischung von Trialkylaluminium und Alkyltitanat gegeben wird. Dies ist beispielsweise in US4532370A beschrieben. Alternative Fahrweisen und Systeme sind bekannt, bei denen überwiegend 2-Butene als Produkt entstehen.

Verschiedene Ausführungsformen der Erfindung sollen nun anhand von Schaltbildern näher erläutert werden. Zum Zwecke der besseren Lesbarkeit und Verständlichkeit sind die Schaltbilder auf das Wesentliche reduziert. Insbesondere wurden Förderorgane und Einrichtungen zum Ändern von Druck und Temperatur nicht eingezeichnet. Es zeigen:
Fig. 1: Schaltbild eines herkömmlichen MTO Prozesses mit Rückführung der C₄-Kohlenwasserstoffe (Stand der Technik);
Fig. 2: Schaltbild eines herkömmlichen MTO Prozesses mit Abschöpfung der C₄-Kohlenwasserstoffe (Stand der Technik);
Fig. 3: Schaltbild einer ersten erfindungsgemäßen Ausführungsform mit einer der Metathese vorgeschalteten Propan-Propen-Kolonne;
Fig. 4: Schaltbild einer zweiten erfindungsgemäßen Ausführungsform mit einer der Metathese nachgeschalteten Propan-Propen-Kolonne;
Fig. 5: Schaltbild einer dritten erfindungsgemäßen Ausführungsform mit einer der Metathese nachgeschalteten Propan-Propen-Kolonne und nur einer einzigen C₄-C₅-Kolonne.

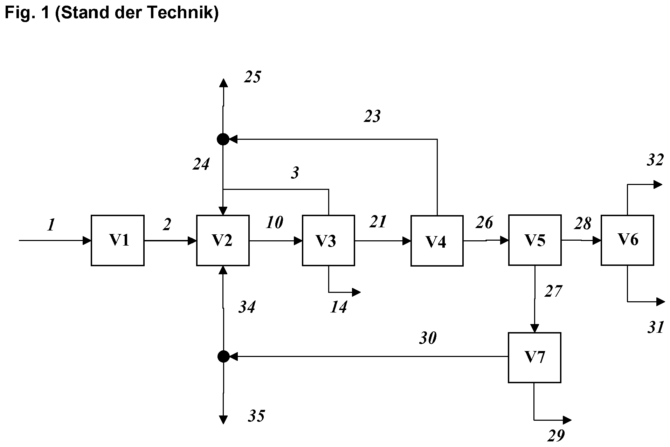

Ein Schaltbild eines herkömmlichen MTP-Prozesses ist in Figur 1 abgebildet. Ein Strom bereitgestellten Methanols (1) wird, nach Erhitzung und Verdampfung, einer ersten Reaktionsstufe (V1) zur Umsetzung des Methanols zu Dimethylether (DME) zugeführt. In einer zweiten Reaktionsstufe (V2) erfolgt die vollständige oder teilweise Umsetzung von DME zu Olefinen. Hierzu wird der erste Reaktionsaustrag (2), der zumindest DME, Methanol und Wasser enthält, nach weiterer Erhitzung auf Reaktionstemperatur der zweiten Reaktionsstufe (V2) zur Umsetzung des DME und Methanols zu Olefinen zugeführt. In die zweiten Reaktionsstufe (V2) werden auch die Ströme (3), (24) und (34) rückgeführt. In der zweiten Reaktionsstufe (V2) erfolgt die Umsetzung des noch vorhandenen Methanols, des DME sowie rezyklierter Komponenten zu Kohlenwasserstoffen. Gegebenenfalls reagiert auch Wasser mit. Der zweite Reaktoraustrag (10), der zumindest DME, Methanol, Wasser und C₁ bis C₆-Kohlenwasserstoffe enthält, wird nach Abkühlung einem Verfahrensschritt (V3) zugeführt, in dem der zweite Reaktionsaustrag (10) gequenscht wird und Wasser sowie eine organische Phase (13), die nicht umgesetztes DME, Methanol und ggf. Restwasser enthält, durch Destillation und Phasentrennung abgetrennt werden. DME, Wasser und Methanol werden als eine organische Fraktion (3) zur zweiten Reaktionsstufe (V2) zurückgeführt und überschüssiges Wasser wird als wässrige Fraktion (14) ausgeschleust. Das übrige Kohlenwasserstoffgemisch (21) wird, nach Verdichtung, in eine Leichtsieder-Kolonne (V4) durch Destillation in eine ethenreiche Leichssieder-Fraktion (23), die überwiegend C₁- und C₂-Kohlenwasserstoffe enthält und in eine schwerer siedende Fraktion (26), die C₃- und höhere Kohlenwasserstoffe enthält, aufgetrennt. Die Leichtsieder-Fraktion (23) wird teilweise in die zweite Reaktionsstufe (V2) zurückgeführt. Um eine unerwünschte Anreicherung von Nebenkomponenten im Prozess zu vermeiden, wird ein Purge-Strom (25) ausgeschleust. Die schwerer siedende Fraktion (26) wird in einer C₃-Kolonne (V5) durch Destillation in eine Fraktion reich an C₃-Kohlenwasserstoffen (28) und einen Strom (27), der C₄-Kohlenwasserstoffe und höhere Kohlenwasserstoffe enthält, aufgetrennt. Die C₃-reiche Fraktion (28) wird in einer Propan/Propen-Kolonne (V6) durch Destillation in eine propenreiche Fraktion (32), der fast reines Propen enthält, und eine propanreiche Fraktion (31), der überwiegend Propan enthält, aufgetrennt. Der Strom (27) wird in einer Hochsieder-Kolonne (V7) durch Destillation in eine leichter siedende Fraktion (30), die überwiegend C₄- und C₅-Kohlenwasserstoffe enthält und in eine Hochsieder-Fraktion (29), die C₅₊-Kohlenwasserstoffe, also C₆- und höhere Kohlenwasserstoffe enthält, aufgetrennt. Die leichter siedende Fraktion (30) wird teilweise in die zweite Reaktionsstufe (V2) zurückgeführt. Um eine unerwünschte Anreicherung von Nebenkomponenten im Prozess zu vermeiden, wird ein Purge-Strom (35) ausgeschleust.

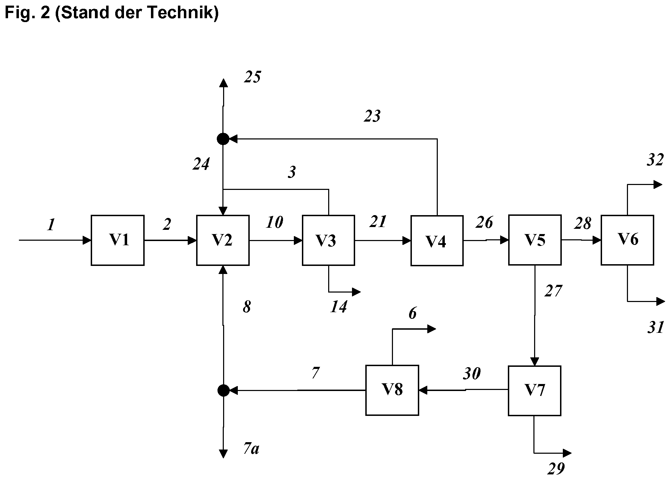

Ein Schaltbild einer Variante eines herkömmlichen MTP-Prozesses ist in Figur 2 dargestellt. In dieser Variante werden die C₄-Kohlenwasserstoffe nicht in die zweite Reaktionsstufe (V2) rezykliert, sondern in einer C₄-Kolonne (V8) von den C₅-Kohlenwasserstoffen (7) abgetrennt und als C₄-reiche Fraktion (6) gewonnen. Diese enthält prozessbedingt eine Mischung und gesättigten und ungesättigten C₄-Isomeren. Die C₄-Ausbeute lässt zu wünschen übrig, da der Prozess auf die Produktion von C₃-Olefinen, nämlich Propen (32), getrimmt ist.

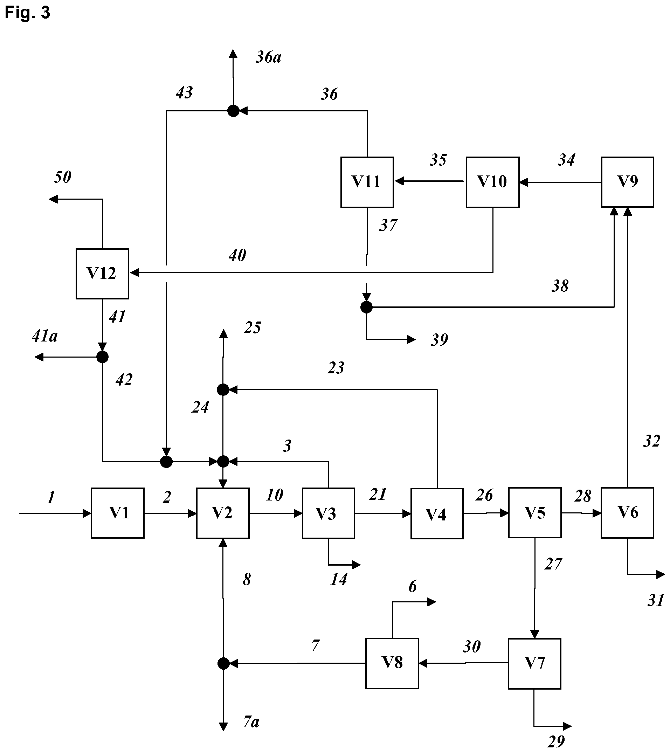

Ein Schaltbild einer ersten erfindungsgemäßen Ausführungsform einer Anlage, in der das erfindungsgemäße Verfahren durchgeführt werden kann, ist in Fig. 3 dargestellt. Soweit diese Ausführungsform den vorbeschriebenen MTP-Prozess entspricht, wird auf die eingehende Beschreibung der Figuren 1 und 2 verwiesen.

In dieser ersten erfindungsgemäßen Ausführungsform wird eine propenreiche Fraktion (32) in einem dritten Reaktionsschritt (V9) zusammen mit dem Strom (38) durch Metathese ganz oder teilweise zu Butenen und Ethen sowie geringeren Anteilen von Pentenen umgesetzt. Ein drittes Reaktionsgemisch (34) aus der Metathese (V9) wird in einer C₃-Kolonne (V10) durch Destillation in einen Strom (35), der überwiegend Propen, Propan und Ethen enthält, sowie in einen Strom (40), der überwiegend Butene und Pentene enthält, aufgetrennt. In einer C₂-Kolonne (V11) wird von Strom (35) eine ethenreiche Fraktion (36), die überwiegend Ethen enthält, abgetrennt. Dabei fällt eine Fraktion (37) an, der überwiegend Propen und Propan enthält. Diese wird, nach Abtrennung eines Purge-Stroms (39) zur Vermeidung der Anreicherung von Propan auf unerwünschte Konzentrationen, als propenreiche Fraktion in die Metathese (V9) zurückgeführt. Die ethenreiche Fraktion (36) kann, nach Ausschleusung eines Purge-Stroms (36a), teilweise in den zweiten Reaktionsschritt (V2) zurückgeführt werden.

In einer C₅-Kolonne (V12) werden die Butene von höher siedende Komponenten, überwiegende in der Metathese gebildete Pentene, abgetrennt. Die C₅-reiche Fraktion (41) kann ganz oder teilweise in den Verfahrensschritt (V2) zurückgeführt werden. Die butenreiche Fraktion (50) enthält überwiegend lineare Butene (1-Buten und 2-Butene) und stellt zusammen mit Strom (6) das Produkt des erfindungsgemäßen Verfahrens dar. Zur weiteren Erhöhung der Ausbeute an C₄-Kohlenwasserstoffen kann von einer teilweisen Rückführung von Strom (36) abgesehen werden. Stattdessen kann dieser Strom optional einer Ethylendimerisierung zugeführt werden. Die Ethylendimerisierung findet in einer vierten Reaktionsstufe statt, die nicht zeichnerisch dargestellt ist. Nebenprodukte aus der Dimersierung können optional auch wieder in die zweite Reaktionsstufe (V2) rückgeführt werden.

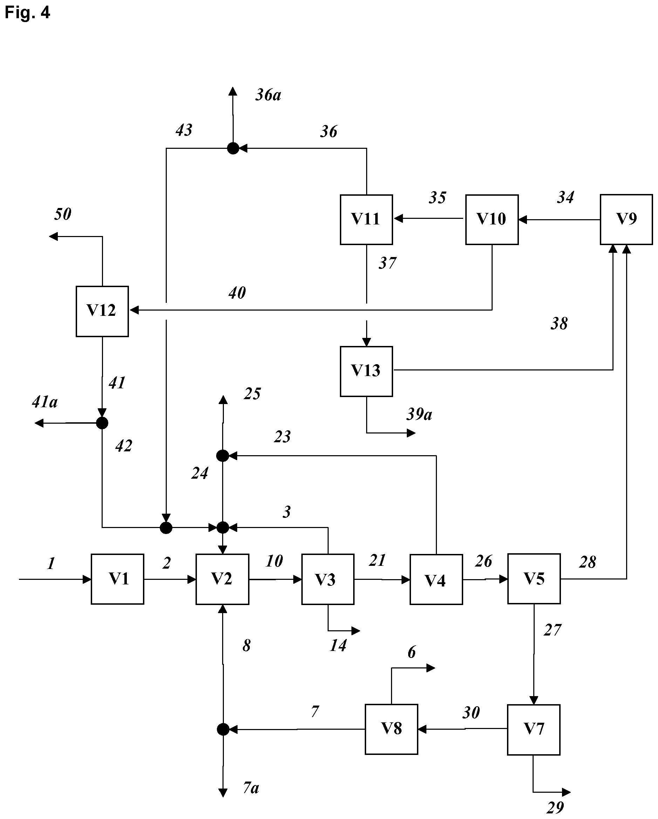

Ein Schaltbild einer zweiten erfindungsgemäßen Ausführungsform einer Anlage, in der das erfindungsgemäße Verfahren durchgeführt werden kann, ist in Figur 4 dargestellt. In dieser Variante wird auf einer teilweisen Abtrennung des Propans (in Verfahrensschritt (V6) in Figur 3) verzichtet. Stattdessen wird eine Propan enthaltende, Propenreiche Fraktion (28) direkt der Metathese (V9) zugeführt und diese mithin in Gegenwart von Propan durchgeführt. In Verfahrensschritt (V13) wird dann durch Destillation aus dem Rückführstrom (37) eine propanreiche Fraktion (39a) abgetrennt. Diese Variante hat gegenüber der Variante in Figur 3 den Vorteil, dass der Aufwand zur Abtrennung des Propans (39a) aus Strom (37) geringer ist als aus Strom (28).

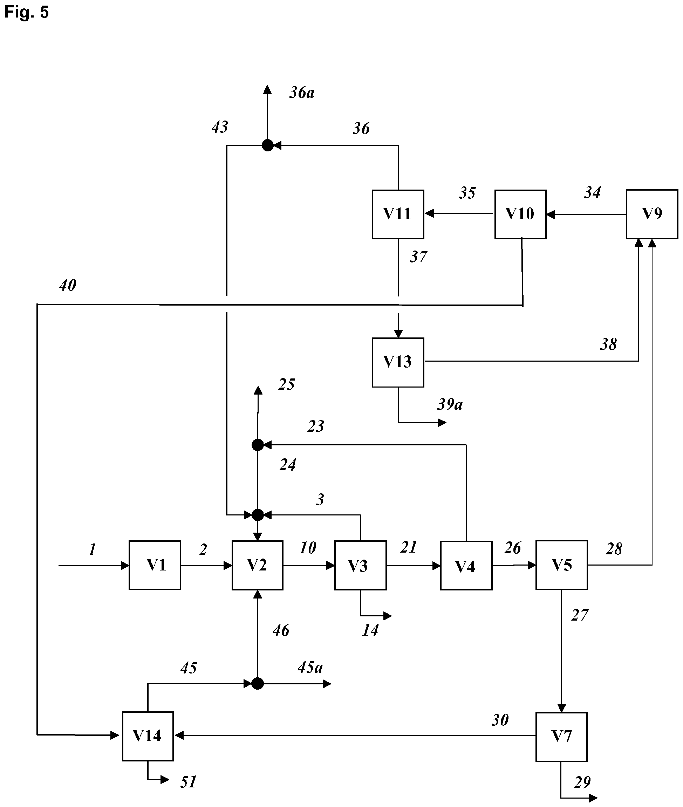

Ein Schaltbild einer dritten erfindungsgemäßen Ausführungsform einer Anlage, in der das erfindungsgemäße Verfahren durchgeführt werden kann, ist in Figur 5 dargestellt. In dieser Variante wird auf die Verfahrensschritte (V8) und (V12) aus den in Figur 3 und 4 gezeigten Ausführungsformen verzichtet. Stattdessen können die C₅-Kohlenwasserstoffe aus den Strömen (30) und (40) in nur einem Verfahrensschritt (V14) vom Zielprodukt, den C₄-Kohlenwasserstoffen, abgetrennt. Diese Variante hat gegenüber den in Figur 3 und 4 gezeigten Varianten den Vorteil, dass der apparative Aufwand zur Aufreinigung der C₄-Kohlenwasserstoffen geringer ist.

### Bezugszeichenliste

- V1:: erste Reaktionsstufe zur Umsetzung von Methanol zu DME (Verfahrensschritt b): DME-Synthese)
- V2:: zweite Reaktionsstufe zur Umsetzung von DME zu Olefinen (Verfahrensschritt c): MTP-Reaktor)
- V3:: Quensche, Wasserabtrennung und Rückführung Wasser, Methanol und DME
- V4:: Leichtsieder-Kolonne (Abtrennung von C₂-Kohlenwasserstoffen und leichter siedenden Komponenten)
- V5:: C₃-Kolonne (Abtrennung von C₃-Kohlenwasserstoffen)
- V6:: Propan-Propen-Kolonne
- V7:: Hochsieder-Kolonne (Abtrennung von C₅₊-Kohlenwasserstoffen und höher siedenden Komponenten)
- V8:: C₄-Kolonne
- V9:: dritte Reaktionsstufe zur Umsetzung von Propen in Olefine (Verfahrensschritt e): Metathese-Reaktion)
- V10:: C₃-Kolonne (Abtrennung von C₃-Kohlenwasserstoffen und leichter siedenden Komponenten)
- V11:: C₂-Kolonne
- V12:: C₅-Kolonne
- V13:: Propan-Propen-Trennung
- V14:: C₄-C₅-Trennung
- 1:: Methanol
- 2:: erstes Reaktionsgemisch aus V1, Mischung aus u.a. Methanol, DME, Wasser
- 3:: Rückführstrom in V2, Mischung aus u.a. Methanol, DME, Wasser
- 6:: C₄-reiche Fraktion enthaltend u.a. 1-Buten, 2-Butene, n-Butan
- 7:: C₅-Kohlenwasserstoffe
- 7a:: C₅-Kohlenwasserstoffe
- 8:: C₅-Kohlenwasserstoffe
- 10:: zweites Reaktionsgemisch aus V2, Mischung u.a. aus Methanol, DME, Wasser, Kohlenwasserstoffen
- 14:: Wasseraussschleusung (Quensche)
- 21:: Kohlenwasserstoffgemisch, u.a. C₁-C₆-Kohlenwasserstoffe
- 23:: Leichtsiederfraktion enthaltend u.a. Methan, Ethen, Ethan
- 24:: Kohlenwasserstoffgemisch, u.a. Methan, Ethen, Ethan
- 25:: Purge-Strom aus Leichtsiedern, u.a. Methan, Ethen, Ethan
- 26:: schwerer siedende Fraktion enthaltend u.a. C₃-C₆-Kohlenwasserstoffe
- 27:: Kohlenwasserstoffgemisch, u.a. C₄-C₆-Kohlenwasserstoffe
- 28:: C₃- Kohlenwasserstoffe (Propen, Propan)
- 29:: Hochsiederfraktion, C₅₊-Kohlenwasserstoffe und höher siedende Komponenten
- 30:: Kohlenwasserstoffgemisch, u.a. C₄-C₅-Kohlenwasserstoffe
- 31:: Propanreiche Fraktion
- 32:: Propenreiche Fraktion
- 34:: drittes Reaktionsgemisch aus V9, u.a. Ethen, Propan, Propen, Butene, Pentene
- 35:: Purge aus Ethen, Propan, Propen
- 36:: Ethenreiche Fraktion
- 36a:: Purge-Strom aus Ethen
- 43:: Ethen
- 37:: Propan, Propen
- 38:: Propan, Propen
- 39:: Propan, Propen
- 39a:: Propanreiche Fraktion
- 40:: Butene, Pentene
- 41:: C₅-reiche Fraktion enthaltend u.a. Petene
- 41a:: Purge aus Petenen
- 42:: Petene
- 45:: C₅-Kohlenwasserstoffe
- 46:: C₅-Kohlenwasserstoffe
- 47:: C₅-Kohlenwasserstoffe
- 50:: Butenreiche Fraktion (Zielfraktion)
- 51:: Butene

## Patentansprüche

1. Verfahren zur Herstellung von linearen Butenen aus Methanol, umfassend die folgenden Schritte:
a) Bereitstellen von Methanol;
b) Umsetzen des bereitgestellten Methanols in einer ersten Reaktionsstufe in ein erstes Reaktionsgemisch enthaltend Dimethylether, Wasser und gegebenenfalls unumgesetzes Methanol;
c) Umsetzen von Dimethylether in einer zweiten Reaktionsstufe in ein zweites Reaktionsgemisch enthaltend Propen sowie weitere Kohlenwasserstoffe mit zwei, vier und fünf Kohlenstoffatomen, wobei die zweite Reaktionsstufe zumindest teilweise mit dem ersten Reaktionsgemisch gespeist wird;
d) Aufarbeiten des zweiten Reaktionsgemisches unter Erhalt einer propenreichen Fraktion sowie mindestens einer propenarmen Fraktion,
e) Umsetzen von Propen in einer dritten Reaktionsstufe in ein drittes Reaktionsgemisch enthaltend Ethen sowie lineare Butene ausgewählt aus der Gruppe umfassend 1-Buten, cis-2-Buten, trans-2-Buten, wobei die dritte Reaktionsstufe zumindest teilweise mit oder aus der propenreichen Fraktion gespeist wird;
f) Aufarbeiten des dritten Reaktionsgemisches in eine Zielfraktion reich an linearen Butenen und in eine ethenreiche Fraktion,
**dadurch gekennzeichnet,**
**dass** die propenarme Fraktion teilweise in die zweite Reaktionsstufe zurückgefahren wird,
**dass** die propenreiche Fraktion Propan enthält, dass die Umsetzung in der dritten Reaktionsstufe in der Gegenwart von Propan erfolgt und dass im Zuge der Aufarbeitung des dritten Reaktionsgemischs eine propanreiche Fraktion gewonnen wird.

2. Verfahren nach Anspruch 1, **dadurch gekennzeichnet, dass** die ethenreiche Fraktion zumindest teilweise in die zweite Reaktionsstufe zurückgeführt wird.

3. Verfahren nach einem der Ansprüche 1 oder 2, **gekennzeichnet durch** den Schritt
g) Umsetzen von Ethen in einer vierten Reaktionsstufe in ein viertes Reaktionsgemisch umfassend lineare Butene ausgewählt aus der Gruppe umfassend 1-Buten, cis-2-Buten, trans-2-Buten, wobei die vierte Reaktionsstufe aus der ethenreichen Fraktion gespeist wird.

4. Verfahren nach einem der Ansprüche 1 bis 3, **dadurch gekennzeichnet, dass** im Zuge der Aufarbeitung des zweiten Reaktionsgemisches des Weiteren eine Fraktion reich an Kohlenwasserstoffen mit zwei Kohlenstoffatomen, eine Fraktion reich an Kohlenwasserstoffen mit vier Kohlenstoffatomen und eine Fraktion reich an Kohlenwasserstoffen mit fünf Kohlenstoffatomen gewonnen werden, wobei die Fraktion reich an Kohlenwasserstoffen mit zwei Kohlenstoffatomen und die Fraktion reich an Kohlenwasserstoffen mit fünf Kohlenstoffatomen zumindest teilweise zurück in die zweite Reaktionsstufe gefahren werden.

5. Verfahren nach einem der Ansprüche 1 bis 4, **dadurch gekennzeichnet, dass** im Zuge der Aufarbeitung des zweiten Reaktionsgemisches des Weiteren eine Hochsiederfraktion gewonnen wird, welche Kohlenwasserstoffe mit mehr als fünf Kohlenstoffatomen enthält.

6. Verfahren nach einem der Ansprüche 1 bis 5, **dadurch gekennzeichnet, dass** im Zuge der Aufarbeitung des zweiten Reaktionsgemisches des Weiteren eine wässrige Fraktion gewonnen wird.

7. Verfahren nach einem der Ansprüche 1 bis 6, **dadurch gekennzeichnet, dass** die Bereitstellung des Methanols die folgenden Schritte umfasst:
h) Bereitstellen einer wasserstoffhaltigen oder wasserstofffreien Kohlenstoffquelle;
i) Herstellen von Synthesegas enthaltend Kohlenstoffmonoxid und Wasserstoff aus der Kohlenstoffquelle; erforderlichenfalls unter Zugabe von Wasser bzw. Wasserdampf;
j) katalytisches Umsetzen des Synthesegases in Methanol in einer fünften Reaktionsstufe.

8. Verfahren nach Anspruch 7, **dadurch gekennzeichnet**, es eine fossile oder eine nachwachsende Kohlenstoffquelle oder eine Mischung aus beidem eingesetzt wird, insbesondere ausgewählt aus der Gruppe umfassend: Steinkohle, Braunkohle, Erdölfraktionen, Torf, Erdgas, Ölsand, Shale-Gas, Holz, Biogas, Biomasse, Müll, Mist, Klärschlamm.

9. Verfahren nach einem der vorgehenden Ansprüche, **dadurch gekennzeichnet, dass** die Umsetzung in der ersten Reaktionsstufe in Gegenwart eines festen Silica-Alumina-Kataysators erfolgt, bevozugt, dass sie in der Dampfphase erfolgt.

10. Verfahren nach einem der vorgehenden Ansprüche, **dadurch gekennzeichnet, dass** die Umsetzung in der zweiten Reaktionsstufe in Gegenwart eines ZeolithKatalysators erfolgt.

11. Verfahren nach einem der vorgehenden Ansprüche, **dadurch gekennzeichnet, dass** die Umsetzung in der dritten Reaktionsstufe in Gegenwart eines Wolfram und/oder Molybdän-Katalysators erfolgt.

12. Verfahren nach Anspruch 11, **dadurch gekennzeichnet, dass** in der dritten Reaktionsstufe nicht umgesetztes Propen aus dem dritten Reaktionsgemisch abgetrennt und in die dritte Reaktionsstufe zurückgeführt wird.

13. Verfahren nach einem der vorgehenden Ansprüche, **dadurch gekennzeichnet, dass** die Umsetzung in der vierten Reaktionsstufe in Gegenwart eines katalytischen Systems aus Trialkylaluminium und Alkyltitanat in Ethern, vorzugsweise in Tetrahydrofuran erfolgt.

## Claims

1. Process for preparing linear butenes from methanol, which comprises the following steps:
a) provision of methanol;
b) reaction of the provided methanol in a first reaction stage to give a first reaction mixture containing dimethyl ether, water and possibly unreacted methanol;
c) reaction of dimethyl ether in a second reaction stage to give a second reaction mixture containing propene and also further hydrocarbons having two, four and five carbon atoms, where the second reaction stage is at least partly supplied with the first reaction mixture;
d) work-up of the second reaction mixture to give a propene-rich fraction and at least one low-propene fraction,
e) reaction of propene in a third reaction stage to give a third reaction mixture containing ethene and linear butenes selected from the group consisting of 1-butene, cis-2-butene, trans-2-butene, where the third reaction stage is supplied at least partly with or from the propene-rich fraction;
f) work-up of the third reaction mixture to give a target fraction rich in linear butenes and an ethene-rich fraction,
**characterized**
**in that** the low-propene fraction is partly recirculated to the second reaction stage,
**in that** the propene-rich fraction contains propane, in that the reaction in the third reaction stage occurs in the presence of propane and in that a propane-rich fraction is isolated during the course of the work-up of the third reaction mixture.

2. Process according to Claim 1, **characterized in that** the ethene-rich fraction is at least partly recirculated to the second reaction stage.

3. Process according to either of Claims 1 and 2, **characterized by** the step
g) conversion of ethene into a fourth reaction mixture comprising linear butenes selected from the group consisting of 1-butene, cis-2-butene, trans-2-butene in a fourth reaction stage, where the fourth reaction stage is supplied from the ethene-rich fraction.

4. Process according to any of Claims 1 to 3, **characterized in that** a fraction rich in hydrocarbons having two carbon atoms, a fraction rich in hydrocarbons having four carbon atoms and a fraction rich in hydrocarbons having five carbon atoms are also isolated during the course of work-up of the second reaction mixture, where the fraction rich in hydrocarbons having two carbon atoms and the fraction rich in hydrocarbons having five carbon atoms are at least partly recirculated to the second reaction stage.

5. Process according to any of Claims 1 to 4, **characterized in that** a high boiler fraction containing hydrocarbons having more than five carbon atoms is also isolated during the course of the work-up of the second reaction mixture.

6. Process according to any of Claims 1 to 5, **characterized in that** an aqueous fraction is also isolated during the course of the work-up of the second reaction mixture.

7. Process according to any of Claims 1 to 6, **characterized in that** the provision of the methanol comprises the following steps:
h) provision of a water-containing or water-free carbon source;
i) preparation of synthesis gas containing carbon monoxide and hydrogen from the carbon source; if necessary with addition of water or water vapour;
j) catalytic conversion of the synthesis gas into methanol in a fifth reaction stage.

8. Process according to Claim 7, **characterized in that** a fossil carbon source or a renewable carbon source or a mixture of the two is used, in particular the carbon source selected from the group consisting of: hard coal, brown coal, petroleum fractions, peat, natural gas, oil sand, shale gas, wood, biogas, biomass, domestic waste, manure, sewage sludge.

9. Process according to any of the preceding claims, **characterized in that** the reaction in the first reaction stage occurs in the presence of a solid silica-alumina catalyst, preferably **in that** it occurs in the vapour phase.

10. Process according to any of the preceding claims, **characterized in that** the reaction in the second reaction stage occurs in the presence of a zeolite catalyst.

11. Process according to any of the preceding claims, **characterized in that** the reaction in the third reaction stage occurs in the presence of a tungsten and/or molybdenum catalyst.

12. Process according to Claim 11, **characterized in that** propene which has not reacted in the third reaction stage is separated off from the third reaction mixture and recirculated to the third reaction stage.

13. Process according to any of the preceding claims, **characterized in that** the reaction in the fourth reaction stage occurs in the presence of a catalytic system composed of trialkylaluminium and alkyl titanate in ethers, preferably in tetrahydrofuran.

## Revendications

1. Procédé de fabrication de butènes linéaires à partir de méthanol, comprenant les étapes suivantes :
a) la préparation de méthanol ;
b) la transformation du méthanol préparé dans une première étape de réaction en un premier mélange réactionnel contenant de l'éther diméthylique, de l'eau et éventuellement du méthanol non réagi ;
c) la transformation de l'éther diméthylique dans une deuxième étape de réaction en un deuxième mélange réactionnel contenant du propène, ainsi que d'autres hydrocarbures à deux, quatre et cinq atomes de carbone, la deuxième étape de réaction étant au moins partiellement alimentée avec le premier mélange réactionnel ;
d) le traitement du deuxième mélange réactionnel pour obtenir une fraction riche en propène et au moins une fraction pauvre en propène,
e) la transformation du propène dans une troisième étape de réaction en un troisième mélange réactionnel contenant de l'éthène, ainsi que des butènes linéaires choisis dans le groupe comprenant le 1-butène, le cis-2-butène, le trans-2-butène, la troisième étape de réaction étant au moins partiellement alimentée avec ou à partir de la fraction riche en propène ;
f) le traitement du troisième mélange réactionnel en une fraction cible riche en butènes linéaires et en une fraction riche en éthène,
**caractérisé en ce que**
la fraction pauvre en propène est partiellement recyclée dans la deuxième étape de réaction,
**en ce que** la fraction riche en propène contient du propane, **en ce que** la réaction dans la troisième étape de réaction a lieu en présence de propane et **en ce qu'**au cours du traitement du troisième mélange réactionnel, une fraction riche en propane est obtenue.

2. Procédé selon la revendication 1, **caractérisé en ce que** la fraction riche en éthène est au moins partiellement recyclée dans la deuxième étape de réaction.

3. Procédé selon l'une quelconque des revendications 1 ou 2, **caractérisé par** l'étape suivante :
g) la transformation de l'éthène dans une quatrième étape de réaction en un quatrième mélange réactionnel comprenant des butènes linéaires choisis dans le groupe comprenant le 1-butène, le cis-2-butène, le trans-2-butène, la quatrième étape de réaction étant alimentée à partir de la fraction riche en éthène.

4. Procédé selon l'une quelconque des revendications 1 à 3, **caractérisé en ce qu'**au cours du traitement du deuxième mélange réactionnel, une fraction riche en hydrocarbures à deux atomes de carbone, une fraction riche en hydrocarbures à quatre atomes de carbone et une fraction riche en hydrocarbures à cinq atomes de carbone sont en outre obtenues, la fraction riche en hydrocarbures à deux atomes de carbone et la fraction riche en hydrocarbures à cinq atomes de carbone étant au moins partiellement recyclées dans la deuxième étape de réaction.

5. Procédé selon l'une quelconque des revendications 1 à 4, **caractérisé en ce qu'**au cours du traitement du deuxième mélange réactionnel, une fraction de composants de point d'ébullition élevé est en outre obtenue, qui contient des hydrocarbures contenant plus de cinq atomes de carbone.

6. Procédé selon l'une quelconque des revendications 1 à 5, **caractérisé en ce qu'**au cours du traitement du deuxième mélange réactionnel, une fraction aqueuse est en outre obtenue.

7. Procédé selon l'une quelconque des revendications 1 à 6, **caractérisé en ce que** la préparation du méthanol comprend les étapes suivantes :
h) la préparation d'une source de carbone contenant de l'hydrogène ou sans hydrogène ;
i) la fabrication d'un gaz de synthèse contenant du monoxyde de carbone et de l'hydrogène à partir de la source de carbone ; si nécessaire avec ajout d'eau ou de vapeur d'eau ;
j) la transformation catalytique du gaz de synthèse en méthanol dans une cinquième étape de réaction.

8. Procédé selon la revendication 7, **caractérisé en ce qu'**une source de carbone fossile ou renouvelable ou un mélange des deux est utilisé, notamment choisie dans le groupe comprenant : la houille, le lignite, les fractions de pétrole, la tourbe, le gaz naturel, le sable pétrolifère, le gaz de schiste, le bois, le biogaz, la biomasse, les déchets, le fumier, les boues d'épuration.

9. Procédé selon l'une quelconque des revendications précédentes, **caractérisé en ce que** la transformation dans la première étape de réaction a lieu en présence d'un catalyseur de silice-alumine solide, de préférence dans la phase gazeuse.

10. Procédé selon l'une quelconque des revendications précédentes, **caractérisé en ce que** la transformation dans la deuxième étape de réaction a lieu en présence d'un catalyseur de zéolithe.

11. Procédé selon l'une quelconque des revendications précédentes, **caractérisé en ce que** la transformation dans la troisième étape de réaction a lieu en présence d'un catalyseur de tungstène et/ou de molybdène.

12. Procédé selon la revendication 11, **caractérisé en ce que** le propène non réagi dans la troisième étape de réaction est séparé du troisième mélange réactionnel et recyclé dans la troisième étape de réaction.

13. Procédé selon l'une quelconque des revendications précédentes, **caractérisé en ce que** la transformation dans la quatrième étape de réaction a lieu en présence d'un système catalytique constitué de trialkylaluminium et de titanate d'alkyle dans des éthers, de préférence dans du tétrahydrofurane.
